# EUROPÄISCHE PATENTANMELDUNG

(11) **EP 1 905 473 A2**
(43) Veröffentlichungstag der Anmeldung: **02.04.2008**
(21) Anmeldenummer: 07118831.2
(22) Anmeldetag: 19.08.2005
(51) Int. Cl.: A61M 15/00

(54) **Inhalator für pulverförmige, insbesondere medizinische Substanzen**

(30) Priorität: 27.08.2004 DE 102004041524
(62) Teilanmeldung aus: 05786868.9
(71) Anmelder: von Schuckmann, Alfred, 47627 Kevelaer (DE)
(72) Erfinder: von Schuckmann, Alfred, 47627 Kevelaer (DE)
(74) Vertreter: Rieder, Hans-Joachim

(57) **Zusammenfassung**

Die Erfindung betrifft einen Inhalator (1) für pulverförmige, insbesondere medizinische, Substanzen mit einem zu einem Mundstück (3) führenden Saugluftkanal (12), ferner einer Vorratskammer (11) für die Substanz (10) und einer bewegten Dosierkammer (26) zum Abteilen einer bestimmten Substanzmenge (10') aus der Vorratskammer (11) und Verbringen einer Substanzmenge (10') in eine Bereitschaftsstellung (Fig. 4) zur Übergabe an den Saugluftstrom (S). Um einen gattungsgemäßen Inhalator, insbesondere im Hinblick auf die Beladung der Dosierkammer in vorteilhafter Weise weiterzubilden, wird vorgeschlagen, dass die Dosierkammer (26) in der Bereitschaftsstellung mit einem relativ zu dem Tauchschieber (26) beweglichen Verschlusskolben (52) verschlossen ist, welcher abhängig von einem Saugunterdruck in eine Entleerungs-Freigabestellung (Fig. 5) bewegbar ist.

## Beschreibung

Die Erfindung betrifft einen Inhalator für pulverförmige, insbesondere medizinische, Substanzen gemäß Gattungsbegriff des Hauptanspruches.

Ein Inhalator der in Rede stehenden Art ist aus der US-PS 5429122 bekannt. Dort wird die ringförmige Dosierkammer bei Abnahme einer Verschluss-Steckkappe durch Federkraft hochbewegt und in den Bereich eines Saugluftstromes gelegt. Wird nicht schnellstens und mit voller Kraft angesaugt, können sogar Teilmengen der Substanz in den Luftstromsaugkanal fallen, evtl. sogar schon beim federnden Hochschnellen der Dosierkammer.

Aufgabe der Erfindung ist es, einen gattungsgemäßen Inhalator, insbesondere im Hinblick auf die beladene Dosierkammer in vorteilhafter Weise weiterzubilden.

Diese Aufgabe ist zunächst und im Wesentlichen durch den Gegenstand des Anspruchs 1 gelöst, wobei darauf abgestellt ist, dass die Dosierkammer in eine verschlossene Entnahme-Bereitschaftsstellung tritt, welche Verschlussstellung erst vom - genügend starken - Saugluftstrom geöffnet wird. Diese steht nach schraubengangförmiger Verlagerung der Dosierkammer in die Übergabeposition erst dann zur Übergabe an den Saugluftstrom und damit einhergehender Inhalation bereit, wenn tatsächlich der genügend starke Saugluftstrom vom Inhalierenden aufgebracht ist. Das wirkt auch einer Entmischung der meist aus mehreren Komponenten bestehenden Substanz entgegen.

Die Gegenstände der weiteren Ansprüche sind nachstehend im Bezug zu dem Gegenstand des Anspruchs 1 erläutert. So wird weiter vorgeschlagen, dass die Dosierkammer in einem als Flachteil ausgeformten Tauchschieber ausgebildet ist. Dies gestattet eine gute Verschlussstellung des Verschlusskolbens bei noch genügender Verschiebbarkeit. Die Dosierkammer ist hierbei weiter bevorzugt als Querbohrung in dem als Flachteil ausgeformten Tauchschieber gestaltet. Als besonders vorteilhaft erweist sich hierbei, wenn der Tauchschieber Verschlusskappen-abhängig verlagerbar ist, so dass in Ausübung der gewohnten Handhabung zum Schließen und Öffnen durch Verschlusskappendrehung zugleich eine Beladung der Dosierkammer und eine lineare, sowie überlagerte rotatorische Bewegung der Dosierkammer in die verschlossene Übergabeposition erreicht wird. Eine besonders wirksame Maßnahme ergibt sich durch eine konische Querbohrung zur Ausformung der Dosierkammer. Das den Tauchschieber ausformende Flachteil weist im Querschnitt ein Kantenverhältnis von etwa 1:2 bis 1:5 auf. Durch die flachteilartige Ausformung des Tauchschiebers ist ein Dreh-Auflockerungseffekt im Zentrumsbereich der in der in der Vorratskammer bevorrateten Substanz erreicht, was zugleich das Eintauchen des Tauchschiebers in den Pulversee und die vollständige Füllung begünstigt. Der Verschlusskolben ist zu dem Tauchschieber relativ linear beweglich und entsprechend von dem als Flachteil ausgeformten Tauchschieber durchsetzt, was weiter die zueinander drehfeste aber reibungsarme Anordnung von Verschlusskolben und Tauchschieber bringt. Die Anordnung, dass dem Ende größeren lichten Durchmessers der Dosierkammer ein Luftdurchlass kleineren Durchmessers als dieser zugeordnet ist und dem Ende kleineren lichten Durchmessers ein Luftdurchlass größeren Durchmessers als dieser, erzeugt von der weitenden Seite her einen größeren Unterdruck. Sodann bringt die Erfindung in Vorschlag, dass die Luftdurchlässe an einem topfförmigen, den Dorn führenden Drehteil ausgebildet sind und mit Lufteinlässen der Mantelwand eines Mundstückes in Strömungsverbindung stehen. Die entsprechenden Lufteinlässe sind mantelwandseitig des Inhalators so platziert, dass sie weder von den Lippen des Benutzers zugehalten werden können, noch durch die Greifhand, die den stabförmigen Körper des Inhalators umfasst. Eine Minimierung der Gefahr eines Zuhaltens ist überdies durch die Ausbildung mehrerer, voneinander beabstandeter Lufteinlässe gegeben. Im Sinne einer guten Verteilung der pulverförmigen Substanz an den Saugluftstrom ist weiter so vorgegangen, dass die Luftdurchlässe axial versetzt zu den dem Mundstück näherliegenden Lufteinlässen angeordnet sind. Das ergibt beim Öffnen einen zunächst gegenläufigen Strömungsweg. Zudem erweist es sich als vorteilhaft, dass das Drehteil mit seinem Topfboden die Decke der Vorratskammer bildet, dessen Zentrum eine Führungsöffnung für den als Tauchschieber fungierenden Dorn aufweist. Der Topfboden erhält so eine Doppelfunktion: Mittel- oder unmittelbarer Deckel und Führungsloch für den Dorn. Ferner besteht ein vorteilhaftes Merkmal darin, dass der in Tauchrichtung endseitig schraubendreherklingenartig angespitzte Dorn über radiale Flügel mit dem Drehteil drehverbunden ist. Zum einen ist durch die so erzielte schneidenartige Klinge neben einem wirksamen Dreh-Auflockerungseffekt im Zentrumsbereich zugleich das Eintauchen des Dornes in den Pulversee begünstigt und zum anderen besteht eine willkommene Abstützung des Dornes gegenüber dem Drehteil, und was weiter die Ausrichtung der Luftdurchlässe auf die Dosierkammer beibehaltbar macht. Die notwendige lineare Relativbewegung von Dorn und Drehteil zueinander wird mit einfachen Mitteln dadurch erreicht, dass die Topfwand des topfförmigen Drehteils axiale Führungsschlitze aufweist, in denen sich die Flügel führen. Diese Lösung ist weiter gekennzeichnet durch einen vom Mundstück gestellten Zugbegrenzungsanschlag des Dornes, definierend die Entleerungsbereitschaftsstellung der Dosierkammer, die mit ihrem Basiswandabschnitt die Übergabestelle stellt. Die Verschlusskappen-abhängige Lagerung des Dornes ist weiter gekennzeichnet durch eine mundstückseitig liegende, bei Überlast ausklinkende Andockstelle zwischen Dorn und Verschlusskappe. Beim Wiederschließen des Inhalators ergibt sich im Gegenzug das erneute Andocken zwischen Dorn und Verschlusskappe. Eine Ausgestaltung eigenständiger Bedeutung verkörpert sich sodann darin, dass das Drehteil einen Rotor aufweist, dem ein Stator zugeordnet ist, mit bei Rückdrehen des Drehteils in die Dosierkammer eintragend wirkendem Schaufeleffekt. Hierüber ist das Nachbringen und auch die Dichte der Pulvermenge in der Dosierkammer gleich haltbar. Hinzu kommt ein im Umfeld gegebener Auflockerungseffekt, der das Stocken von Pulverpartien ausschließt. Rückdrehen meint Abschrauben der Verschlusskappe und die damit zusammenhängende Ladewirkung der bzw. auf die Dosierkammer. Im Einzelnen ist das Schaufelwerk durch von einer Ringscheibe des Bodens des Drehteils ausgehenden, Steg getragenen Rotorblättern gebildet. Endlich wird vorgeschlagen, dass die Verschlusskappe als Schraubkappe ausgebildet ist und mit dem Mundstück über Drehmitnahmemittel zusammenwirkt. Letztere sind klauenkupplungsartig und treten bei Gewindetrennung außer Eingriff.

(Hieran schließt sich an Seite 4, Zeile 1, bis Seite 15, Zeile 23, der unsprünglichen Beschreibung aus WO 2006/021546.)

Nachstehend ist die Erfindung der beigefügten Zeichnungen, welche lediglich ein Ausführungsbeispiel darstellt, näher erläutert. Es zeigt:
- Fig.1: den erfindungsgemäßen Inhalator in Seitenansicht, vergrößert, in kappenverschlossener Grundstellung;
- Fig. 2: den Vertikalschnitt hierzu;
- Fig. 3: eine Herausvergrößerung aus Fig. 2, den Bereich einer Dosiervorrichtung betreffend;
- Fig. 4: eine Schnittdarstellung gemäß Fig. 2, jedoch bei abgenommener Verschlusskappe und hierdurch bedingter Verlagerung der Dosierkammer in die Endnahme-Bereitschaftstellung;
- Fig. 5: eine der Fig. 4 entsprechende Schnittdarstellung, jedoch eine Stellung im Zuge der Inhalation darstellend;
- Fig. 6: den die Dosierkammer aufweisenden Tauchschieber in einer Einzeldarstellung in Ansicht;
- Fig. 7: die Seitenansicht hierzu;
- Fig. 8: in einer perspektivischen Darstellung den Tauchschieber mit einem zuordbaren Verschlusskolben sowie einer Dichtbuchse.

Dargestellt und beschrieben ist ein Inhalator 1, welcher als bequem mitführbares, kurzstabförmiges Taschengerät realisiert ist, mit einem formbestimmenden abgesetzt, zylindrischen Gehäuse 2.

Das zylindrische, röhrchenartige Gehäuse 2 geht kopfseitig des Inhalators 1 in ein aufgesetztes Mundstück 3 über, welches mundgerecht abgeflacht ist und sich mittels einer becherförmigen Verschlusskappe 4 schützend überfangen lässt. Letztere ist als Schraubkappe realisiert, wozu ein ihr zugeordnetes Innengewinde 5 in ein korrespondierendes Außengewinde 6 an der Mantelwand des Gehäuses 2 eingreift. Im Ansatzbereich des Mundstückes 3 ist der Verschlusskappe 4 mantelaußenwandig ein Clip 7 angeformt.

Fußseitig tritt der Stirnrand der becherförmigen Verschlusskappe 4 anschlagbegrenzend und abdichtend gegen eine Ringschulter 8, welche aufgrund des vorgenannten Absatzes des zylindrischen Gehäuses 2 erzielt ist.

Die Verschlusskappe 4 dient zugleich als Betätigungshandhabe 9 zur Ausbringung einer pulverförmigen Substanz 10 in reproduzierbaren Teilmengen 10', wozu der axiale Schraubhub des Gewindeeingriffs 5/6 genutzt wird. Die Substanz 10 ist in einer Vorratskammer 11 des Gehäuses 2 gegebenenfalls nachfüllbar aufgenommen. Die jeweils eine Teilmenge 10' an eine außerhalb der Vorratskammer 11 liegende Übergangsstelle U fördernde Dosiervorrichtung ist in ihrer Ganzheit mit D bezeichnet.

Bezüglich des dosierfähigen Gutes handelt es sich um eine medizinische pulverförmige Substanz 10. So können beispielsweise saugstromtransportfähige Grundkörper wie Laktose Träger für oberflächenseitig anhaftende, mikronisierte Arzneimittel-Feinpartikel sein.

Der Dosiervorrichtung D ist nachgeschaltet ein sogenannter Dispergierbereich, in welchem durch den Benutzer ein Saugluftstrom S erzeugt wird. Dieser trägt die exakt abgeteilte Teilmenge 10' der Substanz 10 in der Übergabestelle U restfrei ab. Der zum Mundstück 3 leitende Saugluftkanal ist mit dem Bezugszeichen 12 versehen.

Den unteren Abschluss der Vorratskammer 11 bildet ein topfförmiger Druckboden 13, welcher in Richtung des Mundstückes 3 mittels einer Druckfeder 14 unter Federbelastung steht. Die Druckfeder 14 stützt sich mit der fußseitigen Endwindung an einer das Gehäuse 2 dort schließenden Bodenkappe 15 ab. Diese steht in Rasteingriff zum hier querschnittsgrößeren Abschnitt des Gehäuses 2, wobei ein entsprechender Rastkragen 16 der Bodenkappe 15 in eine passende Ringnut des Gehäuses 2 eingreift.

Die kopfseitige Endwindung der vorgespannten Druckfeder 14 wirkt belastend auf eine Innenschulter 17 eines Hohlkolbens 18 der kolbenförmigen Einrichtung 13/18. Wie aus den Darstellungen zu erkennen, ist der gestuft topfförmige Druckboden 13 mit der Innenschulter 17 des Hohlkolbens 18 rastverbunden.

Der Topfrand des Druckbodens 13 stellt eine Ringlippe 19, die aufgrund ihres gummielastischen Materials die Wandung der Vorratskammer 21 verlustfrei abstreift.

Von der Bodenkappe 15 geht zentral ein Hohl-Stehzapfen 20 aus. Dieser bildet zusammen mit dem diesen mit Abstand umgebenden Hohlkolben 18 eine Federkammer 21 für die Druckfeder 14.

Mundstückseitig schließt die Vorratskammer 11 mit einem topfförmigen Drehteil 22 ab, welches mit seinem Topfboden die das Gehäuse 2 überfangende Decke 23 der Vorratskammer 11 bildet.

Im Zentrum der Decke 23 ist eine Führungsöffnung 24 belassen. Diese mittel- oder unmittelbar ausgeführte Führungsöffnung 24 nimmt als Herzstück der Dosiervorrichtung D einen Tauchschieber 25 auf. Dieser fungiert zufolge entsprechender Ausgestaltung als eine bewegte Dosierkammer 26 für die auszuhebende Teilmenge 10', wobei die Bewegung des Tauchschiebers 25 linear in der Längsmittelachse x-x des im Wesentlichen rotationssymmetrisch gestalteten Inhalators 1 erfolgt, überlagert von einer um diese Längsmittelachse x-x durchgeführten Rotationsbewegung. Der Tauchschieber 25 ist im Wesentlichen als Flachteil ausgeformt mit langgestreckt rechteckigem Querschnitt. Das Längenverhältnis von Schmalseite zu Breitseite beträgt in dem dargestellten Ausführungsbeispiel etwa 1:3.

An dem dem Mundstück 3 abgewandten Ende bildet der Tauchschieber 25 eine annähernd schraubendreherklingenartige Zuspitzung aus. Die zwei spiegelsymmetrischen Schrägflanken gehen hierbei von den jeweiligen Breitseiten des Tauchschiebers 25 aus. Das freie schrägflankenbesetzte Ende ist verstumpft.

Die Querschnittsausgestaltung des Tauchschiebers 25 und die Ausspitzung des freien Endbereichs haben aufgrund der Drehmitnahme des Tauchschiebers 25 im Zentrumsbereich auflockernde Wirkung in Bezug auf den See aus pulverförmiger Substanz 10.

Der Hubweg der linear sowie überlagert rotatorisch bewegten Dosierkammer 26 berücksichtigt in beiden Endstellungen des Tauchschiebers 25 ein Zuhalten des Querschnitts der Führungsöffnung 24 mit dosierkammerfüllender Rakel- bzw. Abstreichwirkung über die Länge der besagten Öffnung 24.

Das mundstückseitige Ende der Verschlusskappe 4 bildet eine bei Überlast ausklinkende Andockstelle 28 zwischen Tauchschieber 25 und Verschlusskappe 4. Das verschlusskappenseitige Rastmittel ist dabei ein ausfederfähiger Hakenkranz. Das korrespondierende Ende des Tauchschiebers 25 ist im Querschnitt rotationssymmetrisch ausgeformt, wobei weiter im Übergangsbereich vom Flachteilabschnitt zum zylinderförmigen Endabschnitt ein tellerförmiger Radialkragen 29 auswächst. Mit axialem Abstand zu diesem Radialkragen 29 formt der dem Flachteil abgewandte Endbereich des Tauchschiebers 25 einen Rastkopf 30 aus. Zwischen diesem und dem Radialkragen 29 ist eine wespentaillenartige Ringnut 31 gebildet. In diese greifen einwärts gerichtete Nasen 32 der federnden Zungen des Hakenkranzes ein. Der Rastkopf 30 ist in beiden Richtungen durch die Nasen 32 überwindbar.

Die Nasen 29 bzw. ihre Federzungen sind an einem in eine zentrale Mundstücköffnung 3' ragenden Röhrchen 33 realisiert, welches von der Deckeninnenseite der Verschlusskappe 4, an diesem wurzelnd ausgeht.

Das Mundstück 3 greift über eine Mantelwand 34 verankernd am Hals des Gehäuses 2 an. Diese Verankerung ist mit Bezug auf die Darstellungen unterhalb der Drehteil-Decke 23 in Form einer Raststelle 35 zwischen den beiden Teilen 2,3 ausgebildet. Es kann sich hierbei um eine irreversible Raststelle 35 handeln. Darüber hinaus ist die Decke 23 des Drehteiles 22 durch eine Ringschulter 36 der Mantelwand 34 abgestützt überfangen.

Die zentrale Öffnung 3' des Mundstückes 3 ist im Bereich eines im Wesentlichen in Überkopfstellung angeordneten, topfförmigen Dispergierteils 37 ausgebildet. Dies durch zentrale Durchsetzung des Dispergierteil-Bodens 38. Das in Richtung auf das Drehteil 22 sich hin öffnende Dispergierteil 37 weist eine Topfwandung 39 auf, mit einem Außendurchmesser, welcher dem Außendurchmesser der Topfwandung 40 des Drehteiles 22 entspricht. Das topfförmige Drehteil 22 und das topfförmige Dispergierteil 37 weisen mit ihren Öffnungen aufeinander zu, wobei das Dispergierteil 37 sich mit seiner freien Ringkante auf der zugeordneten Ringkante der Drehteil-Topfwandung 40 abstützt.

Beide Topfwandungen 39 und 40 sind radial nach innen beabstandet zur Innenwandung der Mantelwand 34. Entsprechend stellt sich jeweils um das Drehteil 22 und um das Dispergierteil 37 ein Ringraum 41 ein.

Der Innendurchmesser der Topfwandung 39 des Dispergierteiles 37 ist an den Außendurchmesser des tellerartigen Radialkragens 29 des Tauchschiebers 25 angepasst. Entsprechend erfährt letzterer in dem topfartigen Dispergierteil 37 eine Führung in Linearrichtung.

Jeweils zu ihren offenen Endbereichen hin erweitern sich die Topfräume sowohl des Drehteils 22 als auch des Dispergierteils 37 nach radial außen unter Materialreduzierung der jeweiligen Topfwandungen 39 und 40. Zufolge dieser Ausgestaltung stellt sich ein radial erweiterter Umströmungsbereich 42 ein.

Mit etwa der Materialstärke des Radialkragens 29 des Tauchschiebers 25 zum Topfboden 38 des Dispergierteils 37 beabstandet sind in der Topfwandung 39 Radialdurchlässe 43 vorgesehen, zur Verbindung des Topfinnenraumes mit dem umlaufenden Ringraum 41. Es können wie dargestellt zwei diametral gegenüberliegende Durchlassöffnungen 43 vorgesehen sein. Alternativ kann auch ein umlaufender, durch Stützstege unterbrochener Durchlassschlitz vorgesehen sein.

Der den Topfboden 38 des Dispergierteils 37 umfassende Austrags-Ringraum 44 ist zu dem in etwa in axialer Verlängerung sich erstreckenden Ringraum 41 durch einen an der Topfwandung 39 radial nach außen abragenden Dichtkragen 45 getrennt, welcher Dichtkragen 45 sich innenseitig der Mantelwandung 34 abstützt. Zufolge dieser Ausgestaltung ist eine definierte Umlenkung des Saugluftkanals 12 aus der zentralen axialen Ausrichtung nach radial außen in den im Wesentlichen axial ausgerichteten Auslass-Ringraum 44 erreicht.

Die axialen Längen von Drehteil 22 und Dispergierteil 37 im Bereich ihrer Topfwandungen 39 und 40 sind so gewählt, dass der pulverschöpfende Tauchhub des Tauchschiebers 25 aus einer Befüllungsebene in der Vorratskammer 11 bis in die Übergabestelle U oberhalb der Decke 23 gewährleistet ist.

Die definierte Entleerungsbereitschaftsstellung der Dosierkammer 26 ergibt sich durch Zugbegrenzungsanschlag des Tauchschiebers 25 im Bereich seines Radialkragens 29 gegen den Topfboden 38 des Dispergierteils 37.

Die Dosierkammer 26 ist als im Wesentlichen senkrecht zur Längsmittelachse x - x verlaufende Querbohrung realisiert.

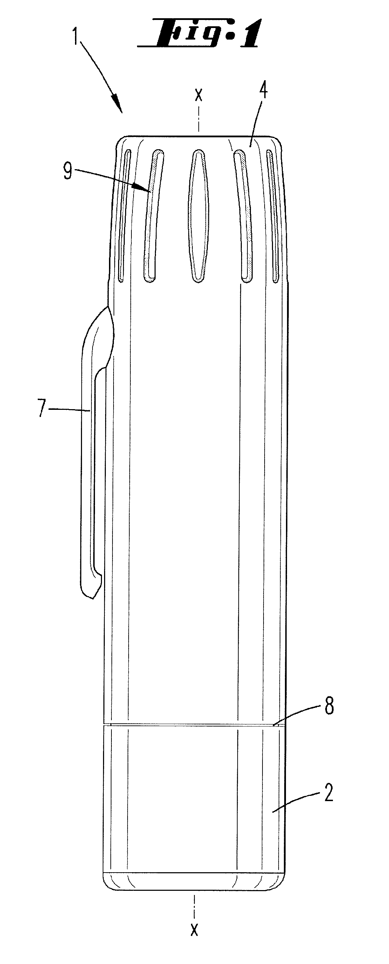

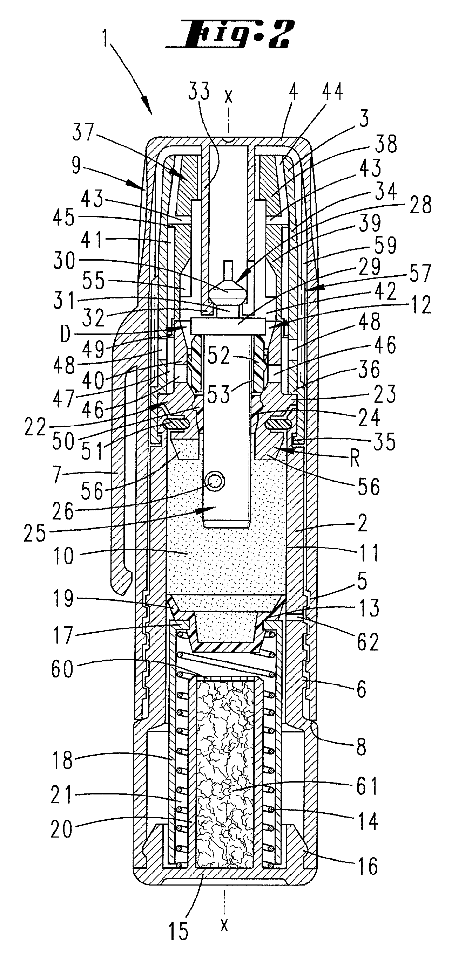

Diese Längsmittelachse x - x bildet zugleich die Rotationsachse. Mit Bezug zu dieser Rotationsachse ist die Dosierkammer 26 exzentrisch angeordnet, so weiter die Breitseiten des als Flachteil ausgeformten Tauchschiebers 25 durchsetzend. Wie insbesondere aus der Darstellung in Figur 2 zu erkennen, ist die Dosierkammer 26 mit Abstand zum freien Ende des Tauchschiebers 25 einer Seitenrandkante der Breitfläche zugeordnet angeordnet.

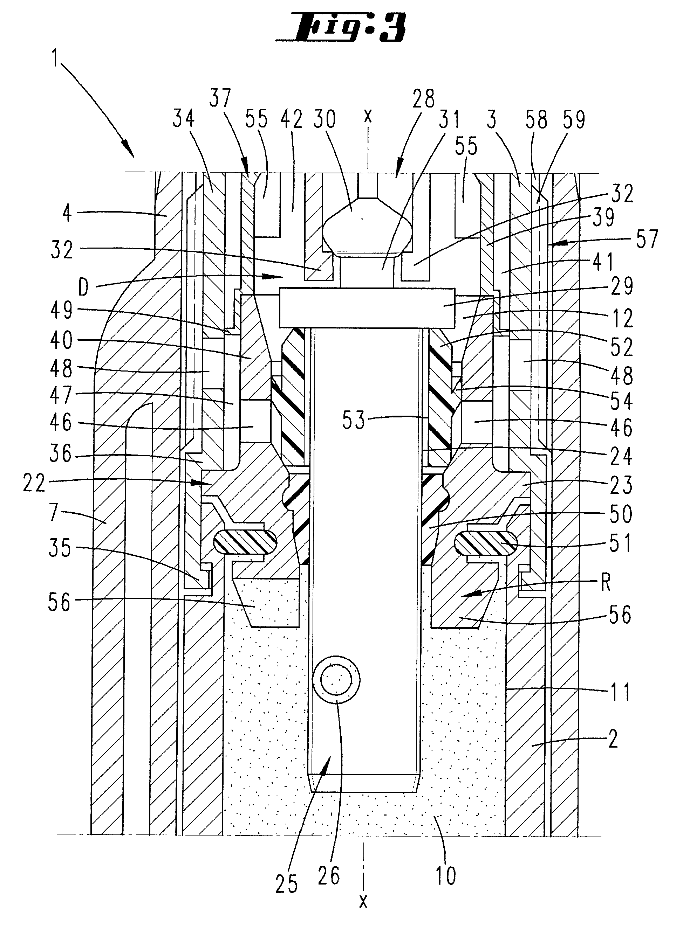

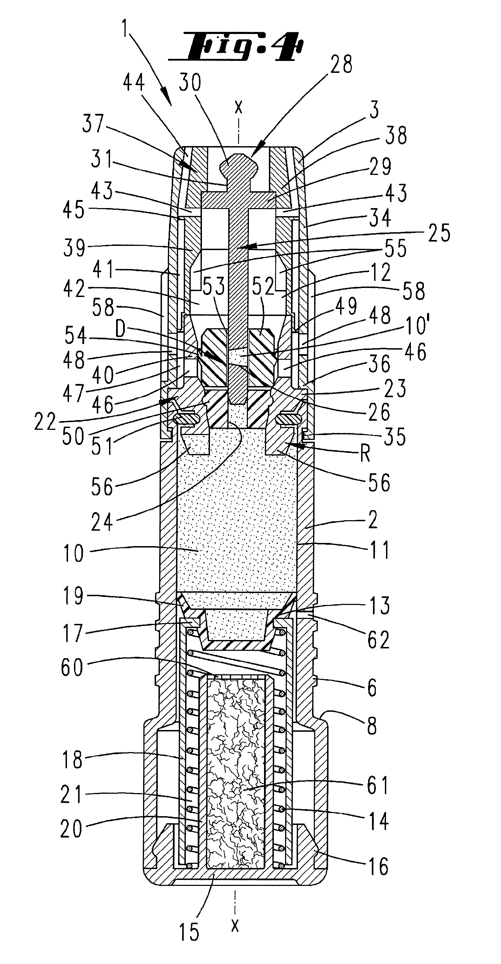

In der Entleerungsbereitschaftsstellung gemäß Figur 4 steht die Dosierkammer 26 im Wirkungsbereich des zentralen Saugluftstromes S. Der Dosierkammer 26 ist ein an den Saugluftkanal 12 anschließender Luftdurchlass 46 zugeordnet, welcher in der Topfwandung 40 des Drehteils 22 ausgebildet ist. Hierbei handelt es sich um radiale Bohrungen, die sich in Bodennähe des topfförmigen Drehteils 22 mit axialem Abstand oberhalb der Oberseite der Decke 23 erstrecken.

Beiden offenen Enden der Dosierkammer 26 ist ein solcher Luftdurchlass 46 mit radialem Abstand vorgelagert. In diesem Zusammenhang kann eine Vorkehrung darin bestehen, dass dem Ende größeren lichten Durchmessers der von einer konischen Querbohrung gebildeten Dosierkammer 26 ein Luftdurchlass 40 kleineren Durchmessers als dieser zugeordnet ist und dem Ende kleineren lichteren Durchmesser der Dosierkammer 26 ein Luftdurchlass 46 größeren Durchmessers als dieser. Hierdurch kann sich hinter dem Luftdurchlass 46 kleineren Durchmessers ein größerer Unterdruck mit vorrangiger Austragswirkung bezüglich der dargebotenen Teilmenge 10' ergeben. Gleichwohl findet der Austrag, das heißt die Entleerung der Dosierkammer 26 von beiden Enden her statt. In den Zeichnungen ist eine Lösung dargestellt, bei welcher die Luftdurchlässe 46 gleiche Durchmesser aufweisen.

Die am topfförmigen, den Tauchschieber 25 führenden Drehteil 22 ausgebildeten Luftdurchlässe 46 sind über einen rückwärtigen Einström-Ringraum 47 radial beabstandet noch mit Lufteinlässen 48 strömungsverbunden. Auch diese sind als Bohrungen ausgeführt und stellen den Anschluss an die Atmosphäre. Der Einström-Ringraum 47 ist zwischen der Außenseite der Topfwandung 40 des topfförmigen Drehteils 22 und der Innenseite der Mantelwand 34 des Mundstücks 3 ausgeformt, dies in axialer Verlängerung zum beschriebenen Ringraum 41. Ein abgestufter, nach radial außen ragender Dichtkragen 49 an dem der Topföffnung des Dispergierteils 37 zugewandten Ende dient der Trennung der Ringräume zueinander sowie zur radialen Ausrichtung des Dispergierteils 37 unter Abstützung an der Innenseite der Mantelwand 34. Die Dichtkragen 49 und 45 des Dispergierteils 37 verhindern einen Strömungskurzschluss zwischen den Lufteinlässen 48 und dem Auslass-Ringraum 44 im Bereich des Mundstückes 3.

Die Luftdurchlässe 46 sind axial versetzt zu den Lufteirlässen 48 angeordnet, wobei letztere dem Mundstück 3 näher liegen. Die beschriebene räumliche Abstandslage führt zu einer zunächst gegenläufigen Einströmung eingesaugter Luft mit Anschluss an den Haupt-Saugluftstrom S.

Die Führungsöffnung 24 für den Tauchschieber 25 ist abstreifend ausgebildet, zufolge dessen auch kein an dem Tauchschiebermantel etwa anhaftendes Pulvergut dosisverfälschend mitgeschleppt wird. Die Führungsöffnung 24 ist nicht unmittelbar vom Drehteil 22 gebildet, sondern durch eine diese Durchtrittsstelle auskleidende Dichtbuchse 50. Letztere besteht aus gummielastischem Material und ist in die Decke 23 über Rastmittel eingeklipst gehalten.

Zwischen dem Drehteil 22 und dem die Vorratskammer 11 bildenden Gehäuse 2 befindet sich gleichfalls ein Dichtelement. Erreicht ist dies durch einen zwischen die Innenwand der Vorratskammer 11 und dem Drehteil 22 eingesetzten Dichtring 51 aus gummielastischem Material. Letzterer ist unter Vorspannung in Ringnuten beider Teile 2, 22 passend eingesetzt. Beide den Dichtring 51 aufnehmende umlaufende Ringnuten weisen eine halbrunde Querschnittsgestaltung auf. Die korrespondierenden Bereiche des Dichtringes 51 sind entsprechend geformt.

Die Dichtbuchse 50 ist drehfest mit dem Drehteil 22 verbunden. Die Führungsöffnung 24 ist angepasst an die Querschnittsausgestaltung des Tauchschiebers 25 gleichfalls langgestreckt rechteckig ausgebildet, zufolge dieses Formschlusses auch der Tauchschieber 25 mit dem Drehteil 22 drehfest verbunden ist.

Mit dem Tauchschieber 25 wirkt ein relativ zu diesem außerhalb der Vorratskammer 11 beweglicher Verschlusskolben 52 zusammen. Dieser kann aus einem gummielastischen Material bestehen und ist zentral durchsetzt vom Flachteilabschnitt des Tauchschiebers 25, wozu der Verschlusskolben 52 eine angepasste, im Grundriss langgestreckt rechteckförmige Lageröffnung 53 besitzt. Diese Lageröffnung 53 ist mit Bezug auf die Querschnittsabmessung des Flachteilabschnittes des Tauchschiebers 25 leicht vergrößert, zufolge dessen eine reibungsarme Verlagerung des Verschlussstopfens 52 auf dem Tauchschieber 25 erreicht ist.

Der Verschlusskolben 52 ist mit einer radial außen umlaufenden Dichtlippe 54 versehen, welche in einer Übergabebereitschaftsstellung gemäß der Darstellung in Figur 4 mit der Innenwand der Drehteil-Topfwandung 40 zusammenwirkt, dies mit Bezug zu der Vorratskammer 11 oberhalb der Luftdurchlässe 46.

In dieser Übergabe-Bereitschaftsstellung liegt der Verschlussstopfen 52 sperrend im Saugluftkanal 12 unter beidseitiger Abdichtung des mit der Teilmenge 10' der Substanz 10 versehenen Dosierkammer 26. Ein Ablegen des Inhalators 1 nach Aktivierung desselben, das heißt nach Verlagerung des Tauchschiebers 25 mit der befüllten Dosierkammer 26 in die Übergabe-Bereitschaftsstellung hat zufolge der Anordnung des Verschlusskolbens 52 nicht den Verlust der abgeteilten Dosiermenge aus der Dosierkammer 26 zur Folge. Der Verschlussstopfen 52 ist selbsthemmend ausgeformt.

Die Verlagerung des Verschlusskolbens 52 ist nur willensbetont möglich, dies in einfachster Weise durch Aktivierung des Saugluftstromes S, das heißt durch üblichen Inhalationssog. In Abhängigkeit von dem hierbei entstehenden Saugunterdruck wird der Verschlusskolben 52 entlang des Flachteilabschnitts des Tauchschiebers 25 nach axial oben verlagert, zur beidseitigen Freigabe der Dosierkammer 26. Der Verschlusskolben 52 wird hierbei in den radial erweiterten Umströmungsbereich 42 bewegt, wobei diese axiale Verlagerung anschlagbegrenzt ist. Hierzu dienen axial ausgerichtete, von der Innenwandung der Dispergierteil-Topfwandung 39 radial nach innen sich erstreckende Anschlagstege 55, gegen welche die ringförmige Dichtlippe 54 des Verschlusskolbens 52 sperrend tritt. Der radiale Abstand der Anschlagstege 55 zueinander entspricht dem Führungsquerschnitt des Dispergierteiles 37 und somit dem Außendurchmesser des tauchschieberseitigen Radialkragens 29.

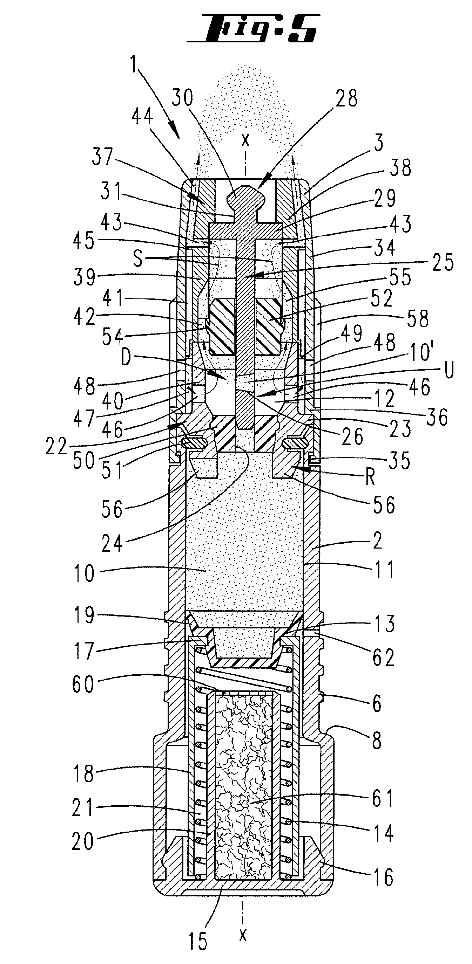

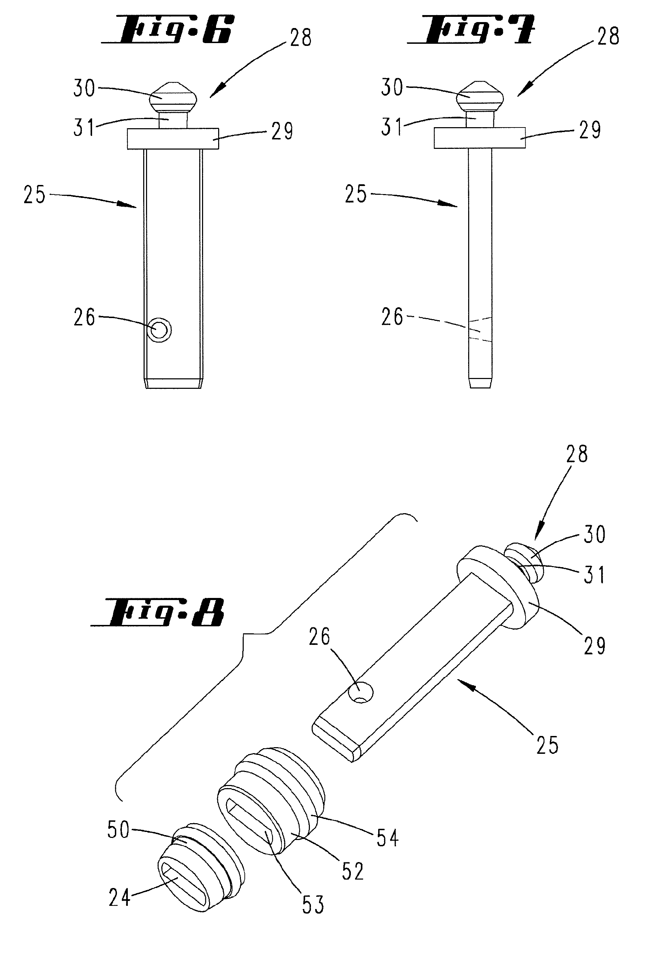

Im Zuge der - wie in Figur 5 schematisch dargestellten - Inhalation wird der Verschlusskolben 52 im Bereich 42 des Mundstückes 3 liegend umströmt.

Förderlich für das Entleeren der Vorratskammer 11 ist die Bereithaltung der pulverförmigen Substanz 10 im Schöpfbereich. Es sind Bedingungen geschaffen, die eine strukturgleiche bzw. homogene Befüllung der Dosierkammer 26 sicherstellen, gespeist aus einem durchgelockerten Umfeld. Hierzu ist vor allem das Drehteil 22 herangezogen. Dieses weist einen im oberen Bereich der Vorratskammer 11 agierenden Rotor R auf. Unter Nutzung der Rotation des Drehteils 22 ergibt sich ein Auflockern der bevorrateten Substanz. Schaufelbildend sind Rotorblätter 56. Diesbezüglich können zwei Rotorblätter 56 vorgesehen sein, welche mit Bezug auf die Längsmittelachse x - x des Inhalators 1 in diametraler Gegenüberlage angeordnet sind. Die vom Boden bzw. der Decke 23 des Drehteils 22 vorratskammerseitig abragenden, freistehenden Rotorblätter 56 sind in einer solchen diametralen Gegenüberlage positioniert, dass sie in Umlaufrichtung genügend beabstandet sind. Geometrisch können diese im Wesentlichen einen Viertelsektor des kreisrunden Querschnitts der Vorratskammer 11 einnehmen.

In Zusammenwirkung mit dem als Flachteil ausgeformten Tauchschieber 25 ist durch die Anordnung der Rotorblätter 56 stets ein gelockertes Umfeld erreicht. Weiter ist durch die zur Rotationsachse des Tauchschiebers 25 exzentrische An ordnung der Dosierkammer 26 eine optimale Befüllung derselben mittels schraubengangförmigen Durchtauchens, durch den Substanzsee erreicht.

Die Drehmitnahme zwischen Mundstück 3 und der sich schraubabhebenden Verschlusskappe 4 erfolgt durch eine Klauenkupplung 57 zwischen beiden. Die besteht aus einer Längszahnung 58 an der Mantelwand 34 des Mundstücks 3, welche Längszahnung 58 in korrespondierende Zahnlücken 59 an der Innenseite der Verschlusskappe 4 eingreifen.

Im Zuge des Schraubabhebens der Verschlusskappe 4 folgt über die Klauenkupplung 57 eine Drehmitnahme des Drehteils 22 und der hierüber mitgeschleppten Teile wie Dichtbuchse 50, Verschlussstopfen 52 und Tauchschieber 25, wobei weiter durch die schraubabhebende Verlagerung der Verschlusskappe 4 zugleich eine axiale Verlagerung des Tauchschiebers 25 über die Andockstelle 28 erfolgt, was eine schraubengangartige Verlagerung der Dosierkammer 26 in die Übergangsstelle U, das heißt in die Übergabe-Bereitschaftsstellung gemäß der Darstellung in Figur 4 bewirkt.

Der Verschlussstopfen 52 verbleibt im Zuge der Linearverlagerung des Tauchschiebers 25 in seiner sich auf der Decke 23 des Drehteils 22 abstützenden, dichtenden Stellung.

Der an der Bodenkappe 15 des Gehäuses 2 wurzelnde Stehzapfen 20 ist endseitig durch einen siebartigen Deckel 60 verschlossen. In dem hierdurch geschaffenen abgegrenzten Raum ist ein Feuchtigkeit absorbierendes Material 61 gefasst.

Der Tauchschieber 25 kann bezüglich des Volumens seiner Dosierkammer 26 variiert werden. Hierzu braucht das Herzstück der Dosiervorrichtung D nämlich der Tauchschieber 25 lediglich ausgetauscht zu werden, um eine andere, genau reproduzierbare Dosierung von Teilmengen 10' zu erzielen.

Der kolbenartig wirkende Druckboden 13 wird in seiner Beweglichkeit gegenüber dem Zylinderraum, welcher vom mittleren Abschnitt des Gehäuses 2 gestellt wird, nicht beeinträchtigt, da das Gehäuse dort eine im Rücken der Ringlippe 19 liegende Luftausgleichsöffnung 62 besitzt.

Der topfförmige Druckboden 13 weist eine zentrale, der Vorratskammer 11 abgewandte Einziehung auf. Diese ist innen von solcher Tiefe, dass der in Grundstellung die Rotorblätter 56 axial nach unten überragende Endabschnitt des Tauchschiebers 25 darin unterkommt.

## Patentansprüche

1. Inhalator (1) für pulverförmige, insbesondere medizinische, Substanzen mit einem zu einem Mundstück (3) führenden Saugluftkanal (12), ferner einer Vorratskammer (11) für die Substanz (10) und einer bewegten Dosierkammer (26) zum Abteilen einer bestimmten Substanzmenge (10') aus der Vorratskammer (11) und Verbringen einer Substanzmenge (10') in eine Bereitschaftsstellung (Fig. 4) zur Übergabe an den Saugluftstrom (S), **dadurch gekennzeichnet, dass** die Dosierkammer (26) in der Bereitschaftsstellung mit einem relativ zu dem Tauchschieber (26) beweglichen Verschlusskolben (52) verschlossen ist, welcher abhängig von einem Saugunterdruck in eine Entleerungs-Freigabestellung (Fig. 5) bewegbar ist.

2. Inhalator nach Anspruch 1, **dadurch gekennzeichnet, dass** der Saugluftkanal (12) oberhalb der Dosierkammer (26) eine Umlenkung nach radial außen aufweist.

3. Inhalator nach Anspruch 1, **dadurch gekennzeichnet, dass** der Tauchschieber (25) als Flachteil ausgeformt ist und die Dosierkammer (26) überlagert rotatorisch beweget und exzentrisch zu einer diesbezüglichen Rotationsachse (x) angeordnet ist.

4. Inhalator nach Anspruch 1, **dadurch gekennzeichnet, dass** der Tauchscheiber (25) mit einem Radialkragen (29) selbst einen Teil der Strömungsumlenkung ausbildet.

5. Inhalator nach einem der Ansprüche 1 bis 4, **dadurch gekennzeichnet, dass** dem Ende größeren lichten Durchmessers der Dosierkammer (26) ein Luftdurchlass (40) kleineren Durchmessers als dieser zugeordnet ist und dem Ende des kleineren lichten Durchmessers ein Luftdurchlass (40) größeren Durchmessers als dieser.

6. Inhalator nach einem der Ansprüche 1 bis 5, **dadurch gekennzeichnet, dass** die Luftdurchlässe (40) an einem topfförmigen, den Dorn (25) führenden Drehteil (22) ausgebildet sind und mit Lufteinlässen (42) der Mantelwand (37) des Mundstückes (3) in Strömungsverbindung stehen.

7. Inhalator nach Anspruch 6, **dadurch gekennzeichnet, dass** die Luftdurchlässe (40) axial versetzt zu den dem Mundstück (3) näherliegenden Lufteinlässen (42) angeordnet sind.

8. Inhalator nach einem der Ansprüche 6 oder 7, **dadurch gekennzeichnet, dass** das Drehteil (22) mit seinem Topfboden die Decke (23) der Vorratskammer (11) bildet, dessen Zentrum eine Führungsöffnung (24) für den als Tauchschieber fungierenden Dorn aufweist.

9. Inhalator nach einem der Ansprüche 6 bis 8, **dadurch gekennzeichnet, dass** der in Tauchrichtung endseitig etwas angespitzte Dorn (25) über radiale Flügel. (33) mit dem Drehteil (22) drehverbunden ist.

10. Inhalator nach Anspruch 9, **dadurch gekennzeichnet, dass** die Topfwand (35) des topfförmigen Drehteils (22) axiale Führungsschlitze (34) aufweist, in denen sich die Flügel (33) führen.

11. Inhalator nach einem der Ansprüche 1 bis 10, **gekennzeichnet durch** einen vom Mundstück (3) gestellten Zugbegrenzungsanschlag (36) des Dornes (25), definierend die Entleerungsbereitschaftsstellung der Dosierkammer (26), die mit ihrem Basiswandabschnitt die Übergabestelle (Ü) stellt.

12. Inhalator nach einem der Ansprüche 2 bis 11, **gekennzeichnet durch** eine mundstückseitig liegende, bei Überlast ausklinkende Andockstelle (28) zwischen Dorn (25) und Verschlusskappe (4).

13. Inhalator nach einem der Ansprüche 6 bis 12, **dadurch gekennzeichnet, dass** das Drehteil (22) einen Rotor (R) aufweist, dem ein Stator (St) zugeordnet ist, mit bei Rückdrehen des Drehteils (22) in die Dosierkammer (26) eintragend wirkendem Schaufeleffekt.

14. Inhalator nach einem der Ansprüche 6 bis 13, **gekennzeichnet durch** von einer Ringscheibe (49) des Bodens des Drehteils (22) ausgehende, steggetragene Rotorblätter (47).

15. Inhalator nach Anspruch 14, **dadurch gekennzeichnet, dass** die Rotorblätter (47) sichelförmigen Umriss aufweisen.

16. Inhalator nach einem der Ansprüche 14 oder 15, **gekennzeichnet durch** zwei einander gegenüberliegende Rotorblätter (47).

17. Inhalator nach einem der Ansprüche 14 bis 16, **dadurch gekennzeichnet, dass** die Rotorblätter (47) sich im Wesentlichen auf einem Viertelsektor erstrecken mit einer radial auf das Zentrum des Dornes (25) gerichteten Flanke (50) und einer etwa rechtwinklig dazu liegenden Schaufelflanke (51) in spaltbelassend tangierender Ausrichtung zum Dorn (25).

18. Inhalator nach Anspruch 17, **dadurch gekennzeichnet, dass** die Flanken (50) in einer gemeinsamen Diametralen (y-y) liegen.

19. Inhalator nach einem der Ansprüche 13 bis 18, **dadurch gekennzeichnet, dass** der Rotor (R) den Stator (St) so untergreift, dass der Stator (St) als von der Innenwand der Vorratskammer (11) radial einwärts abragender, frei in eine Umlaufbahn (54) des Rotors (R) reichender Vorsprung ausgebildet ist.

20. Inhalator nach einem der Ansprüche 13 bis 19, **dadurch gekennzeichnet, dass** der Stator (St) trapezförmigen Umriss besitzt mit Basis in der Innenwand der Vorratskammer (11).

21. Inhalator nach einem der Ansprüche 19 oder 20, **dadurch gekennzeichnet, dass** die Umlaufbahn (54) axial durch die Unterscheibe der Ringscheibe (49) des Drehteils (22) und die ihr zugewandte Innenseite der Rotorblätter (47) begrenzt ist.

22. Inhalator nach einem der Ansprüche 19 bis 21, **dadurch gekennzeichnet, dass** der Stator (St) umrissmäßig unter dem einen Zwischenraum (55) zwischen zwei Rotorblättern (47) belassenden Viertelsektor liegt.

23. Inhalator nach einem der Ansprüche 6 bis 22, **dadurch gekennzeichnet, dass** die Führungsöffnung (24) im Drehteil (22) durch eine den zylindrischen Abschnitt des Dornes (25) umfassende Dichtbuchse (56).

24. Inhalator nach einem der Ansprüche 6 bis 23, **gekennzeichnet durch** einen zwischen Innenwand der Vorratskammer (11) und dem Drehteil (22) mit Vorspannung eingesetzten Dichtring (58).

25. Inhalator nach Anspruch 24, **dadurch gekennzeichnet, dass** der Dichtring (58) in Ringnuten (59, 60) beider Teile passend eingeschnäppert ist, wobei die am Drehteil (22) befindliche Ringnut (59) als V-förmige Kerbnut realisiert und die höhengleich dazu liegende Ringnut (60) der Vorratskammer (11) halbrund ausgebildet ist.

26. Inhalator nach einem der Ansprüche 2 bis 25, **dadurch gekennzeichnet, dass** die Verschlusskappe (4) als Schraubkappe ausgebildet ist und mit dem Mundstück (3) über Drehmitnahmemittel zusammenwirkt.
